(19) Europäisches Patentamt
European Patent Office
Office européen des brevets

(11) **EP 1 009 463 B1**

(12) **EUROPÄISCHE PATENTSCHRIFT**

(45) Veröffentlichungstag und Bekanntmachung des Hinweises auf die Patenterteilung:
**10.12.2003   Patentblatt 2003/50**

(21) Anmeldenummer: **98913554.6**

(22) Anmeldetag: **17.02.1998**

(51) Int Cl.$^7$: **A61M 16/00**

(86) Internationale Anmeldenummer:
**PCT/EP98/00906**

(87) Internationale Veröffentlichungsnummer:
**WO 98/035715 (20.08.1998 Gazette 1998/33)**

(54) **VORRICHTUNG FÜR DAS SCHALTEN IN DIE EIN- ODER AUSATMUNGSPHASE BEI DER CPAP-THERAPIE**

APPARATUS FOR SWITCHING THE INSPIRATION OR EXPIRATION PHASE DURING CPAP THERAPY

APPAREIL POUR FAIRE DEMARRER LA PHASE D'INSPIRATION OU D'EXPIRATION PENDANT UNE THERAPIE PAR VENTILATION SPONTANEE EN PRESSION POSITIVE CONTINUE

(84) Benannte Vertragsstaaten:
**AT BE CH DE DK ES FI FR GB GR IE IT LI LU NL PT SE**

(30) Priorität: **17.02.1997   DE 19706092**

(43) Veröffentlichungstag der Anmeldung:
**21.06.2000   Patentblatt 2000/25**

(73) Patentinhaber: **MAP Medizintechnik für Arzt und Patient GmbH**
**82152 Martinsried (DE)**

(72) Erfinder:
• **VÖGELE, Harald**
**D-81245 München (DE)**
• **GRIEBEL, Jutta**
**D-86922 Pflaumdorf (DE)**
• **MADAUS, Stefan**
**D-81549 München (DE)**

(74) Vertreter: **VOSSIUS & PARTNER**
**Siebertstrasse 4**
**81675 München (DE)**

(56) Entgegenhaltungen:
**EP-A- 0 606 687**          **EP-A- 0 656 216**
**EP-A- 0 714 670**          **EP-A- 0 722 747**
**WO-A-93/08857**

**Beschreibung**

Die Erfindung betrifft eine Vorrichtung für das Umschalten aus der Einatmungsphase in die Ausatmungsphase oder umgekehrt in der CPAP (continous positive airways pressure)-Therapie, bei der fortwährend ein positiver Luftdruck auf die Atmungswege (CPAP-Therapie) ausgeübt wird.

Die CPAP-Therapie dient der pneumatischen Schienung des Kehlkopfes durch kontinuierliche Zufuhr eines positiven Luftdrucks auf die Atemwege. Das Druckniveau wird dabei individuell auf den Patienten abgestimmt. Die Erfindung kommt zum Einsatz bei einem variierten CPAP-Verfahren, wobei das Ausatmen bei einem geringerem Druckniveau als das Einatmen erfolgt. Das hat den Vorteil, daß nicht gegen das hohe Druckniveau ausgeatmet werden muß. Für die Sicherheit des Patienten ist es von großer Bedeutung, daß die Einstellung der unterschiedlichen Druckniveaus beim Übergang von der Ein- zur Ausatmungsphase und umgekehrt mit hoher Genauigkeit erfolgt .

Üblicherweise wird im Stand der Technik der Atemgasfluß vom und zum Patienten gemessen und nach der Zeit differenziert (1. Ableitung), um einen deutlicheren Übergang zwischen der Ein- und der Ausatmungsphase zu erhalten. Die Ableitungen der Flanken der Gasflußkurve werden mit Schwellenwerten verglichen, die einen Übergang in die entsprechende andere Atmungsphase anzeigen. Der Atemgasfluß des Patienten kann jedoch Schwankungen enthalten; ferner können sich auch Einflüsse des Pulsschlages des Patienten im Gasfluß auswirken. Die Folge kann ein Überschwingen der ersten Ableitung sein, wobei der Schwellenwert für die Umschaltung in die andere Atmungsphase vor der erforderlichen Zeit erreicht wird, das andere Druckniveau eingeschaltet wird und die Sicherheit des Patienten gefährdet werden kann.

Aus der EP-A2-0 656 216 ist ein Verfahren für das Schalten in die Einatmungs- oder Ausatmungsphase in der CPAP-Therapie bekannt. Dabei wird ein Generator zur Luftzufuhr für den Patienten für die Bestimmung der Ein- und Ausatmungsphase hinsichtlich der Motorgeschwindigkeit und des Stromverbrauchs überwacht und ein Operationssignal abgeleitet. Aus dem Operationssignal wird ein Signal, das für den Gasfluß zum Patienten repräsentativ ist, abgeleitet. Das nach der Zeit abgeleitete Flußsignal d"flow"/dt wird mit einem ersten und einem zweiten Schwellenwert verglichen und daraus die Änderung der Atmungsphase abgeleitet.

Im EP-A-722 747 wird in einer Teilschaltung die erste zeitliche Ableitung eines Gasflusses während einer Atmungsphase zuerst mit einem hohen Schwellenwert und anschließend mit einem niedrigen Schwellenwert verglichen.

Der Erfindung liegt die Aufgabe zugrunde, eine verbesserte Vorrichtung für das Schalten in die Ein- oder Ausatmungsphase in der CPAP-Therapie zur Verfügung zu stellen, wobei der Übergang in die Ein- oder Ausatmungsphase mit hoher Genauigkeit erkannt und die Sicherheit des Patienten erhöht wird.

Die Aufgabe wird mit den Merkmalen der Patentansprüche gelöst.

Bei der Lösung geht die Erfindung von folgenden Grundgedanken aus.

Die erste Ableitung einer Atemgasflußkurve vom und zum Patienten wird mit zwei aufeinanderfolgenden Schwellenwerten für die Variation der ersten Ableitung verglichen. Dabei ist der erste Schwellenwert größer und daher unempfindlicher. Schwankungen, wie sie oft zu Beginn einer Atemphase auftreten, können diesen hohen Schwellenwert nicht erreichen und somit keine irrtümliche Schaltung eines Druckniveaus der CPAP-Therapie auslösen. Anschließend an den hohen Schwellenwert wird ein niedriger Schwellenwert, der daher empfindlicher ist, festgesetzt, um den Übergang in die nächste Atmungsphase mit hoher Genauigkeit festzustellen. Die Schaltung in die andere Atmungsphase erfolgt, wenn die erste Ableitung den zweiten Schwellenwert erreicht hat.

Bei forcierter Atmung kann auch die hohe Schwelle überschritten werden, daher ist die spontane Atmung jederzeit möglich.

Gemäß der Erfindung wird zusätzlich zum Vergleich mit dem zweiten, niedrigen Schwellenwert in der Ausatmungsphase der zeitliche Verlauf des Gasflusses in mindestens einer Folge von drei Abtastwerten abgetastet. Wenn bei einer derartigen Folge die Bedingung erfüllt ist, daß der zweite und der dritte Abtastwert den jeweils vorangegangenen Abtastwert um einen vorgegebenen Betrag $\Delta$ übersteigen, so erfolgt das Schalten in die Einatmungsphase bei dem dritten Abtastwert; falls diese Umschaltung nicht erfolgt, wird die Schaltung in die Einatmungsphase durch Vergleichen der ersten Ableitung mit dem niedrigen Schwellenwert ausgelöst.

Falls keiner der beiden vorstehend erläuterten Schaltvorgänge in die Einatmungsphase ausgelöst wird, erfolgt die Umschaltung aufgrund einer begrenzenden Zeitschaltung, z.B. spätestens nach 3 bis 4 s für die Ausatmungsphase.

Die Vorteile der Erfindung liegen sowohl in einer geringeren Störanfälligkeit als auch in einer höheren Genauigkeit beim Schalten in die Ein- oder Ausatmungsphase in der CPAP-Therapie.

Im folgenden wird die Erfindung anhand der Zeichnungen näher erläutert. Es zeigen:

Fig . 1 Diagramme zur Erläuterung des erfindungsgemäßen Verfahrens beim Schalten von der Ausatmungsphase in die Einatmungsphase,

Fig. 2 Diagramme zur Erläuterung des erfindungsgemäßen Verfahrens beim Schalten von der Einatmungsphase in die Ausatmungsphase, und

Fig. 3 eine weitere Ausführungsform des erfindungsgemäßen Verfahrens.

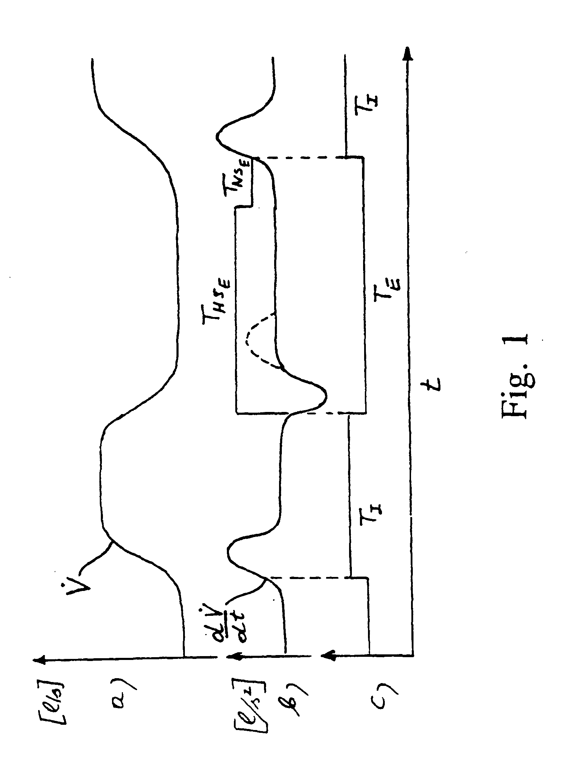

Das Diagramm Fig. 1a) zeigt den zeitlichen Verlauf der Atemgasflußkurve $\dot{V}$ (z.B. in 1/s). Fig. 1b) zeigt die erste Ableitung der Atemgasflußkurve nach der Zeit dV/dt; und Fig. 1c) zeigt einen vergrößerten Ausschnitt der aus der ersten Ableitung abgeleiteten Folge von Einatmungsphase (Inspiration) und Ausatmungsphase (Expiration) $T_I$ bzw $T_E$.

In Fig. 1b) wird der Übergang von der Aus- in die Einatmungsphase bezüglich zweier Schwellenwerte $HS_E$ und $NS_E$ dargestellt. Anschließend an die Schaltung in die Ausatmungsphase $T_E$ wird ein hoher Schwellenwert $HS_E$ für das Auslösen der nächsten Schaltung in die Einatmungsphase festgesetzt. Es ist zu erkennen, daß ein eventuelles Überschwingen (gestrichelte Linie) den Schwellenwert $HS_E$ nicht erreicht und somit keine irrtümliche Schaltung auslösen kann. Nach einer Zeitdauer $T_{HS(E)}$, die vorzugsweise 1,25 mal die Dauer der Einatmungsphase $T_I$ ist, wird ein niedriger Schwellenwert $NS_E$ angesetzt. In diesem Bereich, wo der Übergang in die andere Atmungsphase stattfindet, gewährleistet der niedrige, empfindlichere Schwellenwert eine genaue Bestimmung des Zeitpunkts des Übergangs. Um eine zu lange andauernde Insensibilität eines entsprechenden Geräts zu vermeiden, wird die Dauer der hohen Schwelle $T_{HS(E)}$ in der Ausatmungsphase $T_E$ auf ca. 3 Sekunden begrenzt. Außerdem wird zu Beginn der Ausatmungsphase für eine Zeitdauer von etwa 1 Sekunde die Einatmung verhindert.

Das Festsetzen des niedrigen Schwellenwertes $NS_E$ in der Ausatmungsphase erfolgt nur bei einem negativen Wert des Gasflusses bezüglich einer vorherigen Kalibrierung des Systems auf den Gasfluß "Null". Ist das nicht der Fall, so wird das Inkrafttreten des Schwellenwertes NS um jeweils 100 ms verzögert, bis ein negativer Wert festgestellt wird.

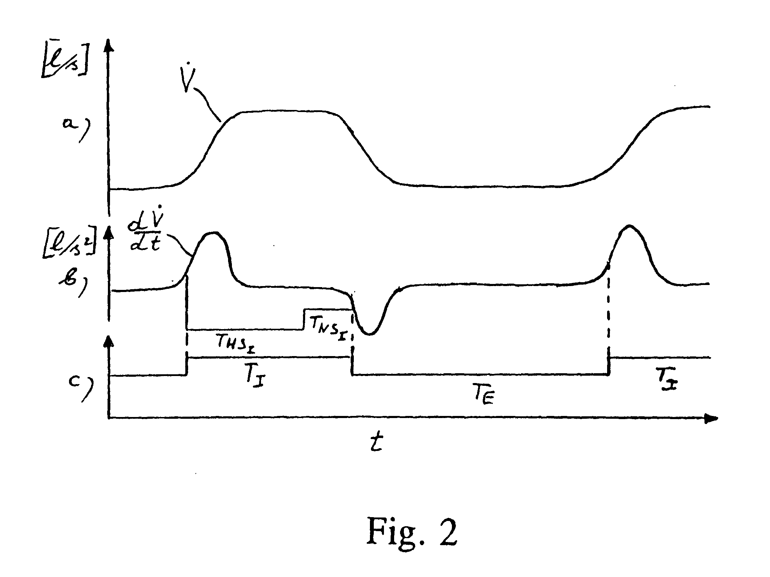

In Fig. 2a), ist der zeitliche Verlauf der Atemgasflußkurve $\dot{V}$ dargestellt; Fig. 2b) zeigt die erste Ableitung der Atemgasflußkurve, und Fig. 2c) zeigt die aus der ersten Ableitung gewonnene Folge der Ein- und Ausatmungsphase.

In Fig. 2 wird der Übergang von der Ein- in die Ausatmungsphase bezüglich der Schwellenwerte $HS_I$ und $NS_I$ dargestellt. Der hohe Schwellenwert $HS_I$ bedeutet das Zulassen einer Abweichung von der ersten Ableitung zu niedrigeren Werten als beim zweiten Schwellenwert $NS_I$. Nach dem Beginn der Einatmungsphase ist also die Schaltung unempfindlicher gegen Abweichungen in der Intensität der ersten Ableitung zu geringeren Werten, die ein unerwünschtes Schalten in die Ausatmungsphase einleiten könnten. Wie in Fig. 1 bewirkt die geringere Abweichung des niedrigen Schwellenwertes von der 1. Ableitung eine erhöhte Empfindlichkeit gegenüber dem Abfallen der Kurve der ersten Ableitung, die den Übergang in die Ausatmungsphase anzeigt. Das Festsetzen des niedrigen Schwellenwertes $NS_I$ in der Einatmungsphase erfolgt nur bei einem positiven Wert des Gasflusses bezüglich einer vorherigen Kalibrierung des Systems auf den Gasfluß "Null".

Ist das nicht der Fall, wird das Inkrafttreten des Schwellenwertes NS um jeweils 100 ms verzögert, bis ein positiver Wert festgestellt wird.

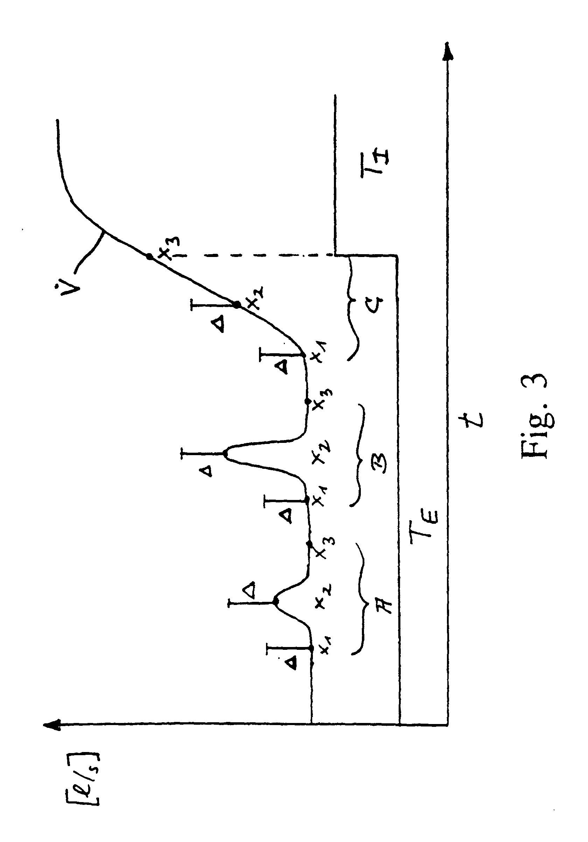

Fig. 3 zeigt eine erfindungsgemäße Ausführungsform, bei der zusätzlich zu dem in Fig. 1 dargestellten Verfahren in der Ausatmungsphase während des Vergleichens der ersten Ableitung mit dem zweiten niedrigen Schwellenwert $NS_E$ die Gasflußkurve $\dot{V}$ in einer Folge von mindestens drei Abtastwerten $X_1$, $X_2$ und $X_3$ abgetastet wird. Für das Schalten in die Einatmungsphase $T_I$ ist dabei die Bedingung gesetzt, daß bei jeweils drei Abtastwerten $X_1$ bis $X_3$ der zweite und der dritte Abtastwert $X_2$ bzw. $X_3$ den jeweils vorangegangenen Abtastwert $X_1$ bzw. $X_2$ jeweils vergrößert um einen festgelegten Betrag $\Delta$ überschreiten müssen; d.h. es müssen die beiden Bedingungen $X_1 + \Delta < X_2$ und $X_2 + \Delta < X_3$ erfüllt sein, wobei $\Delta$ ein geeignet voreingestellter Wert ist. Aus Fig. 3 ist zu erkennen, daß lediglich die letzte Folge C von $X_1$ bis $X_3$-Werten, die auf der Flanke des Einatmungsgasflusses liegt, diese Bedingung erfüllt. Bei der ersten Folge A von $X_1$ bis $X_3$-Werten erfüllt bereits der $X_2$-Wert das genannte Kriterium nicht, und bei der zweiten Folge B von $X_1$ bis $X_3$-Werten wird zwar das Kriterium im Wert $X_2$, jedoch von $X_3$ nicht erfüllt. Auf diese Weise lassen sich kurzzeitige Schwankungen, die z.B. durch den Pulsschlag des Patienten hervorgerufen werden können, und in der ersten Ableitung der Gasflußkurve $\dot{V}$ relativ groß erscheinen, erkennen, so daß das Schalten in die Einatmungsphase durch diese fehlerhaften Signale nicht ausgelöst wird. Durch eine zeitliche Begrenzung der Ausatmungsphase von z.B. 3 bis 4 s wird danach unabhängig von der Schaltauslösung durch das Gasflußsignal oder durch die Ableitung des Gasflußsignals in jedem Fall in die Einatmungsphase umgeschaltet.

Dieses Verfahren führt zum Schalten in die Einatmungsphase auch z.B. in solchen Fällen, wenn eine so flache Atmung vorhanden ist, daß die Werte der Gasflußkurve in der Einatmungsphase den niedrigen Schwellenwert $NS_E$ nicht erreichen.

Die Abtastwerte sollen einen zeitlichen Abstand aufweisen, der größer als die Anstiegsdauer einer Pulswelle des Patienten ist; vorzugsweise beträgt der zeitliche Abstand der Abtastwerte 200 bis 300 ms.

Die Erfindung betrifft eine Vorrichtung, die die in Figuren 1 bis 3 dargestellten Verfahrensschritte ausführen kann.

**Patentansprüche**

1. Vorrichtung zum Schalten in die Ein- oder Ausatmungsphase ($T_I$ bzw. $T_E$) in der CPAP-Therapie, wobei der Atemgasfluß ($\dot{V}$) vom und zum Patienten nach der Zeit differenziert wird, **dadurch gekennzeichnet, daß**

   nach dem Umschalten in die jeweilige Atmungsphase die erste Ableitung (dV/dt) des Gasflusses ($\dot{V}$)

zuerst mit einem ersten, hohen Schwellenwert ($HS_E$, $HS_I$) und anschließend mit einem zweiten, niedrigen Schwellenwert ($NS_E$, $NS_I$) verglichen wird, wobei das Schalten in die jeweilige andere Atmungsphase dann erfolgt, wenn die erste Ableitung den zweiten Schwellenwert ($NS_E$, $NS_I$) erreicht hat, und

daß zusätzlich zum Vergleichen der ersten Ableitung mit dem zweiten niedrigen Schwellenwert ($NS_E$) in der Ausatmungsphase der zeitliche Verlauf des Gasflusses ($\dot{V}$) in einer Folge von mindestens drei Abtastwerten ($X_1$, $X_2$, $X_3$) abgetastet wird, wobei, wenn die beiden Bedingungen

$$X_1 + \Delta < X_2 \text{ und } X_2 + \Delta < X_3$$

$\Delta$ = vorgegebener Zuwachs

erfüllt sind, beim Wert $X_3$ das Schalten in die Einatmungsphase erfolgt, wenn nicht vorher bereits das Schalten durch Vergleichen der ersten Ableitung mit dem niedrigen Schwellenwert ($NS_E$) erfolgte.

2. Vorrichtung nach Anspruch 1, **dadurch gekennzeichnet, daß** die Abtastwerte ($X_1$, $X_2$ und $X_3$) einen zeitlichen Abstand aufweisen, der größer als die Anstiegsdauer einer Pulswelle des Patienten ist.

3. Vorrichtung nach Anspruch 1 oder 2, **dadurch gekennzeichnet, daß** der zeitliche Abstand der Abtastwerte ($X_1$, $X_2$ und $X_3$) bevorzugt 200 bis 300 ms beträgt.

4. Vorrichtung nach einem der Ansprüche 1 bis 3, **dadurch gekennzeichnet, daß** die Zeitdauer ($T_{HS}$) des Vergleichens der ersten Ableitung mit dem hohen Schwellenwert ($HS_E$, $HS_I$) größer als die Zeitdauer ($T_{NS}$) des Vergleichens mit dem niedrigen Schwellenwert ($NS_E$, $NS_I$) ist.

5. Vorrichtung nach Anspruch 4, **dadurch gekennzeichnet, daß** in der Ausatmungsphase gilt:

$$T_I \leq T_{HS(E)} < 4 \text{ s.}$$

6. Vorrichtung nach Anspruch 5, **dadurch gekennzeichnet, daß** bevorzugt

$$T_{HS(E)} \geq 1{,}25 \times T_I$$

ist.

7. Vorrichtung nach einem der Ansprüche 1 bis 6, **dadurch gekennzeichnet, daß** in der Einatmungsphase gilt:

$$1{,}25 \text{ s} \leq T_{HS(I)} < 3 \text{ s.}$$

8. Vorrichtung nach einem der Ansprüche 1 bis 7, **dadurch gekennzeichnet, daß** zu Beginn der Ausatmungsphase eine Einatmung für 1 Sekunde lang verhindert wird.

9. Vorrichtung nach einem der Ansprüche 1 bis 8, **dadurch gekennzeichnet, daß** das Festsetzen des niedrigen Schwellenwertes ($NS_I$) in der Einatmungsphase nur bei einem positiven Wert des Gasflusses ($\dot{V}$) erfolgt und im gegenteiligen Fall das Einsetzen des Schwellenwertes ($NS_I$) um jeweils 100 ms verzögert wird, bis ein positiver Wert festgestellt wird.

10. Vorrichtung nach einem der Ansprüche 1 bis 9, **dadurch gekennzeichnet, daß** das Festsetzen des niedrigen Schwellenwertes ($NS_E$) in der Ausatmungsphase nur bei einem negativen Wert des Gasflusses ($\dot{V}$) erfolgt und im gegenteiligen Fall das Einsetzen des Schwellenwertes ($NS_E$) um jeweils 100 ms verzögert wird, bis ein negativer Wert festgestellt wird.

11. Vorrichtung nach einem der Ansprüche 1 bis 10 wobei die Ausatmungsphase TE zeitlich begrenzt ist.

12. Vorrichtung nach Anspruch 11 wobei die Ausatmungsphase TE auf 3 sec bis 4 sec begrenzt ist.

**Claims**

1. Apparatus for switching into the inhalation or exhalation phase ($T_I$ and $T_E$ respectively) in CPAP therapy, wherein the respiratory gas flow ($\dot{V}$) from and to the patient is differentiated with respect to time, **characterised in that**

after switching over into the respective respiration phase the first derivative ($d\dot{V}/dt$) of the gas flow ($\dot{V}$) is firstly compared to a first high threshold value ($HS_E$, $HS_I$) and then a second low threshold value ($NS_E$, $NS_I$), wherein switching into the respective other respiration phase takes place when the first derivative has reached the second threshold value ($NS_E$, $NS_I$), and

in addition to the comparison of the first derivative to the second low threshold value ($NS_E$) in the exhalation phase the variation in respect of time of the gas flow ($\dot{V}$) is sampled in a sequence of at least three sampling values ($X_1$, $X_2$, $X_3$), wherein if the two conditions

$$X_1 + \Delta < X_2 \text{ and } X_2 + \Delta < X_3 \text{ and}$$

$\Delta$ = predetermined increase

are fulfilled switching into the inhalation phase occurs at the value $X_3$ unless switching was already previously effected by comparison of the first derivative with the low threshold value ($NS_E$).

**2.** Apparatus according to claim 1 **characterised in that** the sampling values ($X_1$, $X_2$, $X_3$) are at a spacing in respect of time which is greater than the rise time of a pulse wave of the patient.

**3.** Apparatus according to claim 1 or claim 2 **characterised in that** the spacing in respect of time of the sampling values ($X_1$, $X_2$ and $X_3$) is preferably 200 to 300 ms.

**4.** Apparatus according to one of claims 1 to 3 **characterised in that** the duration ($T_{HS}$) of the comparison of the first derivative with the high threshold value ($HS_E$, $HS_I$) is greater than the duration ($T_{NS}$) of the comparison with the low threshold value ($NS_E$, $NS_I$).

**5.** Apparatus according to claim 4 **characterised in that** the following applies in the exhalation phase:

$$T_I \leq T_{HS(E)} < 4\text{ s.}$$

**6.** Apparatus according to claim 5 **characterised in that** preferably
$T_{HS(E)} \geq 1.25 \times T_I$.

**7.** Apparatus according to one of claims 1 to 6 **characterised in that** the following applies in the inhalation phase:
$1.25\text{ s} \leq T_{HS(I)} < 3\text{ s.}$

**8.** Apparatus according to one of claims 1 to 7 **characterised in that** an inhalation is prevented for 1 second at the beginning of the exhalation phase.

**9.** Apparatus according to one of claims 1 to 8 **characterised in that** the low threshold value ($NS_I$) in the inhalation phase is fixed only at a positive value in respect of the gas flow ($\dot{V}$) and in the opposite case setting of the threshold value ($NS_I$) is delayed by 100 ms in each case until a positive value is established.

**10.** Apparatus according to one of claims 1 to 9 **characterised in that** the low threshold value ($NS_E$) in the exhalation phase is fixed only at a negative value in respect of the gas flow ($\dot{V}$) and in the opposite case setting of the threshold value ($NS_E$) is delayed by 100 ms in each case until a negative value is established.

**11.** Apparatus according to one of claims 1 to 10 wherein the exhalation phase TE is limited in respect of time.

**12.** Apparatus according to claim 11 wherein the exhalation phase TE is limited to 3 sec to 4 sec.

**Revendications**

**1.** Appareil pour faire démarrer la phase d'inspiration ou d'expiration ($T_I$ ou $T_E$) dans la thérapie CPAP (thérapie par ventilation spontanée en pression positive continue), le flux de gaz respiratoire ($\dot{V}$) venant du patient et allant vers le patient étant différencié d'après le temps, **caractérisé en ce qu'** après le démarrage de la phase de respiration donnée, la première dérivation ($d\dot{V}/dt$) du flux de gaz ($\dot{V}$) est d'abord comparée avec une première valeur de seuil élevée ($HS_E$, $HS_I$) et ensuite avec une deuxième valeur de seuil basse ($NS_E$, $NS_I$), le démarrage de l'autre phase respiratoire donnée se faisant lorsque la première dérivation a atteint la deuxième valeur de seuil ($NS_E$, $NS_I$), et qu'en plus, pour la comparaison de la première dérivation avec la deuxième valeur de seuil basse ($NS_E$) dans la phase d'expiration, l'évolution dans le temps du flux ($\dot{V}$) est palpée en une succession d'au moins trois valeurs de palpage ($X_1$, $X_2$, $X_3$), le démarrage de la phase d'inspiration se faisant à la valeur $X_3$, lorsque les deux conditions
$X_1 + \Delta < X_2$ et $X_2 + \Delta < X_3$
$\Delta$ = accroissement prédéterminé
sont réunies, à condition que le démarrage n'ait pas eu lieu encore avant par la comparaison de la première dérivation avec la valeur de seuil basse ($NS_E$).

**2.** Appareil selon la revendication 1, **caractérisé en ce que** les valeurs de palpage ($X_1$, $X_2$ et $X_3$) présentent un écart dans le temps qui est supérieur à la durée d'augmentation d'une onde du pouls du patient.

**3.** Appareil selon les revendications 1 ou 2, **caractérisé en ce que** l'écart dans le temps des valeurs de palpage ($X_1$, $X_2$ et $X_3$) est de préférence de 200 à 300 ms.

**4.** Appareil selon l'une quelconque des revendications 1 à 3, **caractérisé en ce que** la durée ($T_{HS}$) de la comparaison de la première dérivation avec la valeur de seuil élevée ($HS_E$, $HS_I$) est supérieure à la durée ($T_{NS}$) de la comparaison avec la valeur de seuil basse ($NS_E$, $NS_I$).

**5.** Appareil selon la revendication 4, **caractérisé en ce que** dans la phase d'expiration la formule

$$T_I \text{ est} \leq T_{HS(E)} < 4\text{ s}$$

s'applique.

**6.** Appareil selon la revendication 5, **caractérisé en ce que** la formule

$$T_{HS(E)} \geq 1{,}25 \times T_I$$

s'applique de préférence.

7. Appareil selon l'une quelconque des revendications 1 à 6, **caractérisé en ce que** dans la phase d'inspiration la formule

$$1{,}25 \text{ s} \leq T_{HS(I)} < 3 \text{ s}$$

s'applique.

8. Appareil selon l'une quelconque des revendications 1 à 7, **caractérisé en ce qu'**au début de la phase d'expiration une inspiration est empêchée pendant une seconde.

9. Appareil selon l'une quelconque des revendications 1 à 8, **caractérisé en ce que** la détermination de la valeur de seuil basse ($NS_I$) dans la phase d'inspiration ne se fait que lorsque la valeur du flux de gaz ($\dot{V}$) est positive et dans le cas contraire l'intervention de la valeur de seuil ($NS_I$) est retardée de 100 ms jusqu'à ce qu'une valeur positive soit constatée.

10. Appareil selon l'une quelconque des revendications 1 à 9, **caractérisé en ce que** la détermination de la valeur de seuil basse ($NS_E$) dans la phase d'expiration ne se fait que lorsque la valeur du flux de gaz ($\dot{V}$) est négative et dans le cas contraire l'intervention de la valeur de seuil ($NS_E$) est retardée de 100 ms jusqu'à ce qu'une valeur négative soit constatée.

11. Appareil selon l'une quelconque des revendications 1 à 10, la phase d'expiration TE étant limitée dans le temps.

12. Appareil selon la revendication 11, la phase d'expiration TE étant limitée de 3 secondes à 4 secondes.

Fig. 1

Fig. 2

EP 1 009 463 B1

Fig. 3

EP 1 009 463 B1